(19) Europäisches Patentamt
European Patent Office
Office européen des brevets

(11) **EP 4 799 660 A1**

(12) **EUROPEAN PATENT APPLICATION**

(43) Date of publication:
**02.09.2026 Bulletin 2026/36**

(21) Application number: **25205883.9**

(22) Date of filing: **30.09.2025**

(51) International Patent Classification (IPC):
***A61L 27/46*** *(2006.01)* ***A61L 27/56*** *(2006.01)*

(52) Cooperative Patent Classification (CPC):
(C-Sets available)
**A61L 27/46; A61L 27/56;** A61L 2430/02; A61L 2430/12 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **27.02.2025 CN 202510230892**

(71) Applicant: **WitKang Zhiyuan Medical Devices (Xi'an) Co., Ltd.**
**Xi'an City Shaanxi 710075 (CN)**

(72) Inventors:
- **TIAN, Zhen**
  **Xi'an City, 710075 (CN)**
- **HOU, Jianing**
  **Xi'an City, 710075 (CN)**
- **LI, Yuan**
  **Xi'an City, 710075 (CN)**
- **WANG, Ya**
  **Xi'an City, 710075 (CN)**

(74) Representative: **Habermann, Hruschka & Schnabel**
**Patentanwälte**
**Montgelasstraße 2**
**81679 München (DE)**

Remarks:
The complete document including Reference Table(s) and the Sequence Listing(s) can be downloaded from the EPO website

# (54) USE OF COMPOSITION IN PREPARATION OF BONE REPAIR MATERIAL

(57) The present disclosure provides the use of a composition in the preparation of a bone repair material, wherein, based on 100% by mass of the total dry matter of the composition being 100%, the material comprises: 70%-90% of coralline hydroxyapatite particles, 5%-30% of recombinant human collagen, and 0%-7% of excipient, and the bone repair material is a cancellous bone-mimicking material with a porosity of 85%-99%. The composition provided by the present disclosure has a high porosity and a longitudinal gradient structure, can mimic the properties of natural bone tissue, exhibits superior biocompatibility, biodegradability, hydrophilicity, osteogenic properties, and shape memory function, has excellent clinical convenience, hemostatic and healing-promoting effects, and excellent osteogenic effect, and has wide application prospects in the preparation of bone repair materials for filling and/or repairing bone defects.

a
b
c
d

FIG. 8



(Cont. next page)

(52) Cooperative Patent Classification (CPC): (Cont.)

C-Sets
**A61L 27/46, C08L 89/06**

## Description

TECHNICAL FIELD

The present disclosure belongs to the field of medical materials, and in particular relates to the use of a composition in the preparation of a bone repair material.

BACKGROUND

In the research of artificial bone biomaterials, currently the main categories of the materials include: polymer materials, such as polymethyl methacrylate (PMMA), i.e., bone cement, and high-molecular-weight polyethylene used in artificial joints. These materials exhibit poor biocompatibility and are spaced from bone tissue via fibrous tissue. Inorganic materials are the most widely used materials, mainly ceramic materials, which are divided into bioinert, bioactive, and degradable materials. Bioinert materials include alumina ceramics; bioactive materials include glass ceramics, bioactive glass, hydroxyapatite, etc.; and degradable ceramics are mainly β-tricalcium phosphate (β-TCP). Another class of degradable material not belonging to ceramics is natural coral. The main advantages of bioactive materials are good biocompatibility, the ability to chemically bond with bone tissue or degrade *in vivo,* and high strength. Ceramic materials have a main disadvantage of high brittleness, and their elastic modulus do not easily match that of natural bone, which limits their clinical application to some extent.

Therefore, the development of ideal bone graft substitutes has always been one of the important topics in the field of orthopedic surgery. An ideal bone graft substitute should have the following characteristics: (1) osteoconductivity; (2) osteoinductivity; (3) excellent hydrophilicity; (4) good biocompatibility; (5) degradability, with a degradation rate matching a new bone regeneration rate *in vivo;* (6) appropriate porosity and interconnected pore structure; (7) good mechanical properties; (8) ease of intraoperative handling; (9) ease of sterilization before use; (10) capability of being prepared into a specific size for convenient filling, etc.

Hydroxyapatite (HAP) and collagen (Col protein) are the main inorganic and organic components of natural bone, and both have good biological properties. However, when used individually, they have different drawbacks, failing to meet the requirements of tissue engineering for the properties of biomaterials. By compounding collagen with hydroxyapatite, the adhesive properties of collagen can be effectively utilized to overcome some of the limitations of hydroxyapatite.

SUMMARY

In order to solve the above technical problem, an objective of the present disclosure is to provide the use of a composition in the preparation of a bone repair material.

In order to achieve the above objective, the present disclosure provides a composition for use in the preparation of a bone repair material, wherein, based on 100% by mass of the total dry matter of the composition being 100%, the composition comprises:

70%-90% of coralline hydroxyapatite particles,
5%-30% of a recombinant human collagen, and
0%-7% of an excipient.

The bone repair material is a cancellous bone-mimicking material and has a porosity of 85%-99%.

According to a particular embodiment of the present disclosure, preferably, the porosity of the cancellous bone-mimicking material is 95%-99%.

According to a particular embodiment of the present disclosure, preferably, the bone repair material includes a bone repair material used in filling and/or repair of bone defects.

According to a particular embodiment of the present disclosure, preferably, the bone repair material includes a bone repair material used in dentistry, orthopedics, neurosurgery, and plastic surgery.

According to a particular embodiment of the present disclosure, preferably, the bone repair material used in dentistry includes a bone repair material used in extraction socket filling after tooth extraction or residual root extraction, alveolar ridge restoration, repair of alveolar bone defects caused by periodontal diseases, filling and restoration of dentoalveolar bone defects or bone volume deficiency, and filling of bone defects in maxillofacial non-load-bearing areas.

According to a particular embodiment of the present disclosure, preferably, the bone repair material used in orthopedics includes a bone repair material used in repair of bone defects in non-load-bearing areas, and more preferably includes a bone repair material used in fractures accompanied by bone defects, bone nonunion or malunion and orthopedic correction, benign cystic bone lesions, spinal fusion caused by lumbar instability or lumbar canal stenosis, and bone grafting in joint arthrodesis.

According to a particular embodiment of the present disclosure, preferably, the bone repair material used in neurosurgery includes a bone repair material used in filling of cranial defects caused by craniotomy drilling, filling of cranial defects caused by decompressive craniectomy, filling of bone gaps caused by craniectomy milling cutter, repair of cranial defects caused by intraoperative cranial bone removal, and various other neurosurgical procedures without bone grafting contraindications.

According to a particular embodiment of the present disclosure, preferably, the bone repair material uses in plastic surgery includes a bone repair material used in filling and/or repair of bone defects.

According to a particular embodiment of the present disclosure, the composition is a solid porous material formed by coralline hydroxyapatite particles bonded to each other through a recombinant human collagen.

The recombinant human collagen of the present disclosure is the recombinant human collagen disclosed in CN 108070032 B [METHOD FOR PURIFYING RECOMBINANT HUMAN COLLAGEN].

According to a particular embodiment of the present disclosure, the recombinant human collagen has an amino acid sequence of SEQ ID No: 1.

According to a particular embodiment of the present disclosure, preferably, based on 100% by mass of the total dry matter of the composition being 100%, the material comprises: 70%-90% of coralline hydroxyapatite particles, 5%-23% of recombinant human collagen, and 5%-7% of excipient.

According to a particular embodiment of the present disclosure, the coralline hydroxyapatite particles have a particle size of 0.1 mm-2 mm, a pore size of 50 μm-800 μm, and a porosity of 50%-90%.

According to a particular embodiment of the present disclosure, preferably, the conversion rate of the coralline hydroxyapatite particles is 5%-80%. Preferably, the conversion rate is 5%-30%.

According to a particular embodiment of the present disclosure, preferably, the excipient comprises one of or a combination of two or more of cross-linked porous starch, sodium carboxymethyl cellulose, chitosan, carboxymethyl chitosan, and hydroxypropyl methyl cellulose.

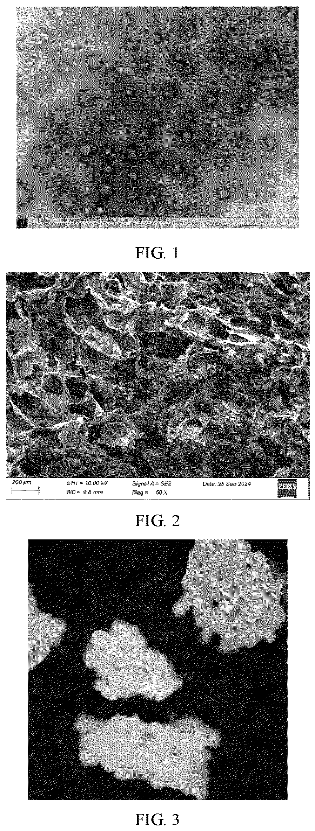

The recombinant human collagen of the present disclosure has regular hydrophilic groups on its exterior, exhibiting ultra-strong aggregation ability, while its interior has hydrophobic groups, forming microscaffolds. The recombinant human collagen of the present disclosure has the self-assembly ability and can undergo self-assembly in hypoxic or vacuum environments. Increasing temperature can enhance the self-assembly ability, and this process does not involve reagent residues caused by chemical cross-linking. The hydrophilicity (structure) of the recombinant human collagen (smart collagen) is as shown in FIG. 1, and the self-assembled structure of the recombinant human collagen under SEM is as shown in FIG. 2.

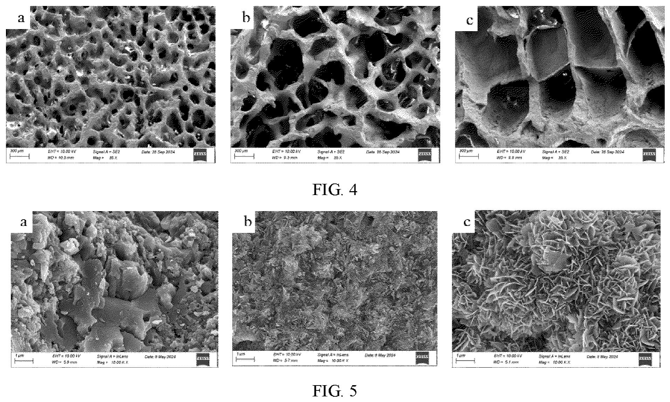

According to a particular embodiment of the present disclosure, the coralline hydroxyapatite particles are prepared by soaking coral stone in a cutting protective agent, performing crushing and granulating, and performing hydrothermal exchange. The appearance of the coral stone of the present disclosure is as shown in FIG. 3, exhibiting a three-dimensional interconnected porous network structure. The microstructure of the coral stone with different pore sizes is as shown in FIG. 4, where FIG. 4a represents dense pores, FIG. 4b represents medium pores, and FIG. 4c represents large pores. The coral stone with medium pores as shown in FIG. 4b most closely resembles the structure of natural bone.

According to a particular embodiment of the present disclosure, the cutting protective agent is a solution containing a polyol.

According to a particular embodiment of the present disclosure, preferably, the polyol is selected from one of or a combination of two or more of glycerol, ethylene glycol, sorbitol, and butanediol.

According to a particular embodiment of the present disclosure, based on the total volume of the cutting protective agent, the volume fraction of the polyol is ≥ 20%.

According to a particular embodiment of the present disclosure, the duration of the soaking is ≥ 3 h.

According to a particular embodiment of the present disclosure, the raw material of the coral stone comprises natural coral and/or artificially cultivated coral.

According to a particular embodiment of the present disclosure, preferably, the natural coral includes *Porites* and/or *Goniopora,* more preferably *Porites.*

According to a particular embodiment of the present disclosure, the step of the hydrothermal exchange comprises: performing immersion with a saturated diammonium hydrogen phosphate solution, and performing a reaction at 0.1-3 Mpa and 150°C-220°C for 6-19 h.

The method for preparing the coralline hydroxyapatite particles of the present disclosure further comprises: subjecting coral to bleaching and washing, then soaking the coral in a cutting protective agent, performing crushing and granulating, and performing hydrothermal exchange to prepare the coralline hydroxyapatite particles.

For the coralline hydroxyapatite particles obtained by the preparation method of the present disclosure, a nanoflower-like hydroxyphosphorylated structure can be formed on their surface, resulting in "nanoflower"-structured coralline hydroxyapatite particles. The surface of these particles exhibits a "nanoflower"-structured morphology under a microscope, as shown in FIG. 5. By soaking in the cutting protective agent, coral can still retain the intact porous structure after being cut and polished into a small particle size, making it more similar to human cancellous bone. The conversion

rate of the "nanoflower"-structured coralline hydroxyapatite can also be controlled by controlling the temperature, time, and the amount of reagent added, thereby preparing coralline hydroxyapatite with different proportions of nanoflowers which can form biomimetic bone structures with different pore sizes with recombinant human collagen, enabling a controllable degradation rate after implantation in the body. The microstructures of the "nanoflower"-structured coralline hydroxyapatite with different conversion rates are as shown in FIG. 5, where FIG. 5a represents an unconverted state, FIG. 5b represents a mildly converted state, and FIG. 5c represents a completely converted state.

For the "nanoflower"-structured coralline hydroxyapatite prepared in the present disclosure, a series of steps such as soaking in a cutting protective agent are performed for treatment, so as to better preserve the pore size and porosity, greatly improving the yield.

According to a particular embodiment of the present disclosure, preferably, the composition has a bone tissue structure and exhibits excellent osteoconductivity and osteoinductivity.

According to a particular embodiment of the present disclosure, the composition is in forms including blocks, flakes, granules, or powder.

According to a particular embodiment of the present disclosure, when the composition is in forms including blocks, flakes, granules, or powder, the porosity is 85%-99%, preferably 90%-99%.

According to a particular embodiment of the present disclosure, when the composition is in the form of a block, the size range may be (1-10) mm × (1-10) mm × (1-10) mm, (10-100) mm × (10-100) mm × (10-100) mm, diameter (2-100) mm × height (5-100) mm, and minor diameter (2-100) mm × major diameter (2-100) mm × height (1-100) mm; for example, the following sizes may be used: 4 mm × 4 mm × 4 mm, 6 mm × 6 mm × 6 mm, 8 mm × 8 mm × 8 mm, 10 mm × 10 mm × 10 mm, 15 mm × 15 mm × 15 mm, 20 cmm × 20 mm × 20 mm, 30 mm × 30 mm × 30 mm, 65 mm × 65 mm × 65 mm, φ 5 mm × height 8 mm, φ 5 mm × height 12 mm, φ 5 mm × height 15 mm, φ 8 mm × height 8 mm, φ 8 mm × height 12 mm, φ 8 mm × height 15 mm, φ 10 mm × height 8 mm, φ 10 mm × height 12 mm, φ 10 mm × height 15 mm, φ 5-8 mm × height 10 mm, φ 6-10 mm × height 10 mm, φ 7-12 mm × height 10 mm, φ 8-15 mm × height 10 mm, φ 5-8 mm × height 15 mm, φ 6-10 mm × height 15 mm, φ 7-12 mm × height 15 mm, φ 8-15 mm × height 15 mm.

According to a particular embodiment of the present disclosure, when the composition is in the form of a sheet, the size range may be (0.1-5) mm × (10-200) mm × (10-200) mm, (0.1-5) mm × (50-200) mm × (50-200) mm, and diameter (5-200) mm × height (1-5) mm; for example, the following sizes may be used: 0.5 mm × 10 mm × 10 mm, 0.5 mm × 15 mm × 15 mm, 0.5 mm × 20 mm × 20 mm, 2 mm × 50 mm × 50 mm, 5 mm × 100 mm × 100 mm, φ 8 mm × height 2 mm, φ 8 mm × height 3 mm, φ 10 mm × height 2 mm, φ 10 mm × height 3 mm, φ 20 mm × height 5 mm, φ 25 mm × height 5 mm, φ 50 mm × height 5 mm, φ 100 mm × height 5 mm.

According to a particular embodiment of the present disclosure, when the composition is in the form of a granule, the particle size distribution range may be 0.18-2 mm; for example, the following distribution ranges may be used: 0.18 mm-0.25 mm 0.25-0.3 mm, 0.3-0.5 mm, 0.5-1 mm, and 1-2 mm.

According to a particular embodiment of the present disclosure, the composition may be prepared in a variety of forms in a mold(s) prior to implantation in a patient.

The composition of the present disclosure may be further subjected to steps such as cutting, trimming, crushing, and sieving according to specific applications to obtain the bone repair material. An aqueous liquid may also be further added to obtain the bone repair material. The aqueous liquid includes blood, water for injection, phosphate buffer, purified water, etc.

In some embodiments, the composition is in the form of a powder, in which the surface of the coralline hydroxyapatite particles is coated with collagen and/or an excipient, which develops adhesion when mixed with a liquid, allowing the composition to be directly used in the powder state for filling at bone defect areas. Alternatively, an aqueous liquid may be added prior to filling to form a moldable paste, thereby obtaining the bone repair material, wherein the paste is injectable and used for filling at bone defect areas.

According to a particular embodiment of the present disclosure, the bone repair material is a filler material of bone tissue and/or a scaffold material of bone tissue.

In another aspect, the present disclosure further provides a method for preparing the above composition, which preparation method comprises:

uniformly mixing "nanoflower"-structured coralline hydroxyapatite particles with a recombinant human collagen solution, and performing freeze-drying to obtain a freeze-dried sample;
subjecting the freeze-dried sample to self-assembly, and performing sterilization to obtain the composition.

In the above preparation method, the mass fraction of the recombinant human collagen in the recombinant human collagen solution is 15%-50%, preferably 20%-50%, and more preferably 30%.

In the above preparation method, preferably, the solvent of the recombinant human collagen solution comprises one of or a combination of two or more of phosphate buffer, purified water, or water for injection.

In the above preparation method, preferably, the recombinant human collagen solution further comprises an

excipient. In the present disclosure, the recombinant human collagen and an excipient may jointly form a collagen scaffold. In the above preparation method, the step of the freeze-drying comprises:

(1) Rapid freezing: process parameters involve reaching a temperature of -80°C to -60°C within 30-240 min;
(2) Pre-freezing: process parameters involve reaching a temperature of -50°C to -30°C within 60-240 min and maintaining the temperature for 120-600 min;
(3) Sublimation: the pre-frozen product is subjected to sublimation under a vacuum degree set at 0.01-0.1 mbar, where the temperature reaches -50°C to -5°C within 10-60 min and then is maintained for 120-600 min;
(4) Vacuum drying: the sublimated product is subjected to vacuum drying under a vacuum degree set at 0.01-0.1 mbar, where the temperature reaches 0°C-40°C within 10-60 min and then is maintained for 120-600 min.

The specific process parameters for freeze-drying in the preparation method of the present disclosure are as shown in Table 1.

Table 1

| Freeze-drying step | | Set temperature (°C) | Set time (min) | Duration (min) | Vacuum degree (mbar) |
|---|---|---|---|---|---|
| 1 | Pre-freezing | -50 to -30 | 60-240 | 120-600 | - |
| 2 | Primary sublimation | -50 to -5 | 10-60 | 120-600 | 0.01-0.1 |
| 3 | Vacuum drying | 0 to 40 | 10-60 | 120-600 | 0.01-0.1 |

In the above preparation method, the pore size structure of the material can be controlled by controlling the freezing rate, where slow freezing results in larger pore size and rapid freezing results in smaller pore size.
The freeze-dried sample of the composition of the present disclosure can undergo self-assembly under low oxygen content or oxygen-free conditions, including three cases: the first case is low-pressure self-assembly; the second case is nitrogen-filled vacuum self-assembly; the third case is oxygen-free self-assembly.
In the above preparation method, the step of the self-assembly comprises:
placing the freeze-dried sample under a vacuum degree of -0.1 to -0.01 MPa or in a nitrogen environment, performing heating to 100°C-300°C, and then maintaining the temperature for 0.5-8 h. Preferably, the temperature of the heating is 100°C-220°C, and the vacuum degree of the nitrogen environment is -0.01 to -100 KPa.
In the above preparation method, preferably, the self-assembly is performed using an electric-heating vacuum drying oven or a precision vacuum nitrogen-filled integrated oven.
In the present disclosure, a recombinant human collagen is proportionally prepared into a recombinant human collagen solution, a collagen scaffold is formed by the freeze-drying and self-assembly processes of the present disclosure, and the porosity of the material is detected using ethanol infiltration or mercury intrusion porosimetry. In the present disclosure, the porosity of the collagen scaffold formed by freeze-drying and self-assembly of the recombinant human collagen solution is ≥ 85%.
In the above preparation method, preferably, the method of the sterilization is radiation sterilization or ethylene oxide sterilization.
In the above preparation method, preferably, the method of the radiation sterilization is cobalt 60 irradiation sterilization and/or electron beam irradiation sterilization, with a sterilization dose of 10-30 kGy.
In the above preparation method, preferably, the sterilization parameters of the ethylene oxide sterilization are: sterilization temperature: 40°C-60°C, sterilization humidity: 40%-60%, ethylene oxide concentration: 400-700 g/m$^3$, vacuum degree: -20-10 KPa, sterilization time: 6-12 h.
In the above preparation method, preferably, the method of the sterilization is electron beam irradiation sterilization, with a sterilization dose of 10-25 kGy.
According to a particular embodiment of the present disclosure, the above preparation method comprises the following steps: (1) preparing "nanoflower"-structured coralline hydroxyapatite by hydrothermal exchange; (2) preparing a recombinant human collagen solution; (3) dispersing the "nanoflower"-structured coralline hydroxyapatite in the recombinant human collagen solution to obtain a suspension; (4) uniformly mixing the recombinant human collagen solution with the suspension of the "nanoflower"-structured coralline hydroxyapatite, then performing rapid freezing, and then performing vacuum freeze-drying to obtain a freeze-dried sample; (5) self-assembling the freeze-dried sample under low oxygen content or oxygen-free conditions; (6) alternatively, crushing and sieving the self-assembled block or sheet samples to prepare granules; (7) finally, sterilizing the block, sheet or granular materials to obtain a composition with a three-dimensional network structure resembling natural bone tissue.
In the present disclosure, the "nanoflower"-structured coralline hydroxyapatite is prepared from natural coral through a series of processes such as hydrothermal exchange, resulting in a unique "nanoflower" structure formed on the

surface of the coral stone. In the preparation of the composition of the present disclosure, the self-assembled recombinant human collagen is mixed with the "nanoflower"-structured coralline hydroxyapatite at a certain mass ratio, and by means of freeze-drying and the biological self-assembly ability of the material, the material with a cancellous bone-mimicking structure and varying pore sizes is prepared. The material has a high porosity and a longitudinal gradient structure to mimic the properties of natural bone tissue.

The composition prepared in the present disclosure exhibits superior biocompatibility, biodegradability, hydrophilicity, osteogenic properties, and shape memory function. The material softens rapidly upon contact with water, showing elasticity and certain flexibility. It can be freely cut according to the shape of the defect area, making it suitable for filling bone defects at any site. Moreover, the composition of the present disclosure utilizes recombinant human collagen, thereby eliminating the inevitable risk of viral contamination associated with conventional animal-derived collagen scaffold materials. No additives are introduced during production, significantly enhancing its safety for use.

In particular, the composition provided by the present disclosure has the following advantages:

1. Good biocompatibility: The "nanoflower"-structured coralline hydroxyapatite, i.e., coralline hydroxyapatite (HAP), exhibits good biocompatibility. Its composition and structure are similar to those of natural bone. Upon implantation in the body, it does not cause systemic or local toxic reactions, nor does it elicit immune rejection. Both the self-assembled recombinant human collagen and the coral stone possess good biocompatibility, and the preparation process mainly involves self-assembly under low oxygen content or oxygen-free conditions, avoiding any chemical reagent residues.
2. Good biodegradability: The self-assembled recombinant human collagen in the composition provided by the present disclosure possesses good biodegradability. The conversion rate of the "nanoflower"-structured coralline hydroxyapatite is controlled by the preparation process, allowing the degradation rate to match the rate of bone tissue growth and repair. The resulting cancellous bone-mimicking material degrades at a rate consistent with the rate of bone tissue ingrowth, enabling complete degradation and perfect integration with bone tissue to form autogenous bone. The bone repair effect is second only to allogeneic bone. It can perfectly integrate with autologous bone within 3-6 months without foreign body sensation. The degradation rate matches the osteogenesis rate. Implantation feeling and CT images indicate that this material can perfectly integrate with autologous bone and eventually develop into autologous bone. In comparison, bone materials prepared from nano-hydroxyapatite and bovine bone cannot be completely degraded. The bone repair effect of bovine bone is inferior to that of allogeneic bone and the cancellous bone-mimicking material of the present disclosure; bovine bone is degraded slowly, with a long bone resorption time, cannot completely integrate with autologous bone, and still exhibits a gritty sensation 3-6 months after re-implantation. It is a "semi-permanent" implant material, and long-term use thereof may cause local osteoporosis or displacement risk. The bone repair effect of nano-hydroxyapatite is superior to that of bovine bone, but inferior to that of allogeneic bone and the cancellous bone-mimicking material of the present disclosure. Additionally, the material is brittle and hard, and difficult to degrade in the human body, potentially existing in the human body for a long time.
3. Excellent mechanical properties and shape memory function: The collagen network provides good mechanical support, giving the product moderate strength and good flexibility.
4. Efficient cell adhesion and proliferation capability: The highly ordered network structure facilitates cell adhesion, proliferation, and migration.
5. Excellent hydrophilicity: The porosity of the composition can reach 98.75% $\pm$ 0.56%. It becomes fully saturated with water or blood within 2-3 seconds, exhibiting excellent hydrophilicity. Good blood supply provides sufficient nutrients and oxygen for bone tissue regeneration while facilitating metabolic waste removal, thereby accelerating the bone healing process. The latest research shows that hydrophilic surfaces can accelerate osseointegration, shorten the waiting time for repair, and significantly improve implant stability. For example, Cowell superhydrophilic implants adopt advanced surface treatment technology to achieve extremely high hydrophilicity. Such surfaces can accelerate bone tissue growth and reduce the incidence of complications, which is particularly important for complex cases with poor periodontal conditions or requiring full-mouth restoration. In addition, the total stability of the hydrophilic implants drops to the lowest point at the 2nd week after implantation, and the ideal osseointegration is achieved at the 4th to 6th weeks, indicating that the hydrophilic surfaces can rapidly initiate the osseointegration process.
6. Biomimetic structure: The composition features a radial gradient structure of natural bone tissue, closely resembling the structure of natural bone tissue in the human body.
7. Excellent clinical convenience: Traditional extraction socket filling materials such as bone powder require membrane coverage to prevent bone powder loss and promote its integration with bone tissue. In contrast, when using the composition of the present disclosure, the extraction socket is mechanically sealed through its network structure, so that infection and further tissue damage are prevented, the need for membrane coverage is eliminated, and the surgical time can be greatly reduced from over 20 minutes in traditional procedures to 2 minutes. Therefore, the bone repair material prepared using the composition of the present disclosure not only simplifies the surgical procedure but also reduces both the treatment time and financial burden for patients.

8. Hemostatic and healing-promoting effects: Due to its good adsorption capacity, the composition of the present disclosure can adsorb and activate platelets, promote blood clot formation, and form thrombus to achieve hemostasis. Simultaneously, the composition exhibits slight volume increase after blood adsorption, generating gentle compression against the bone wall of the extraction socket to achieve tight adaptation, thereby accelerating the healing and osteogenesis processes. In addition, collagen, as a scaffold material, provides sites for cell attachment, promotes cell proliferation and differentiation, and thus facilitates tissue remodeling and healing.

9. Excellent osteogenic effect: The main components of the composition prepared in the present disclosure are hydroxyapatite and collagen, which are the main inorganic and organic components of natural bone. Filling the extraction socket with the composition prepared in the present disclosure can prevent alveolar bone resorption or reduce the extent of alveolar bone resorption, while facilitating creeping coverage of gingival epithelium, thereby benefiting the restoration of the bone height at the extraction site.

## BRIEF DESCRIPTION OF THE DRAWINGS

FIG. 1 shows the hydrophilicity (structure) of the recombinant human collagen (smart collagen).
FIG. 2 shows the self-assembled structure of smart collagen under SEM.
FIG. 3 shows the appearance of coral stone.
FIG. 4 shows the microstructure of coral stone with different pore sizes.
FIG. 5 shows the microstructure of the "nanoflower"-structured coralline hydroxyapatite with different conversion rates.
FIG. 6 shows the appearance and morphology of the compositions (block and sheet) obtained in the examples and comparative examples.
FIG. 7 shows the appearance and morphology of the composition (granular) in Example 2.
FIG. 8 shows the microstructure of the composition in Example 1.
FIG. 9 shows the rehydration state of the compositions in Examples 1 and 5.
FIG. 10 shows the compression deformation experiment results of the composition in Example 1.
FIG. 11 shows the hydrophilic properties and shape memory function of the compositions obtained in the examples and comparative examples.
FIG. 12 shows the cytotoxicity results of the composition in Example 1.
FIG. 13 shows the cell migration results of the composition in Example 1.
FIG. 14 shows the cell proliferation results of the composition in Example 1.
FIG. 15 shows the cell adhesion results of the composition in Example 1.
FIG. 16 shows the *in vitro* degradation results of the composition in Example 1.
FIG. 17 shows imaging of bone repair at rabbit femoral condyle defect using the composition in Example 1.
FIG. 18 shows imaging of bone repair at rabbit femoral condyle defect using the composition in Example 1.
FIG. 19 shows imaging of bone repair in clinical trial (extraction socket filling) using the composition in Example 1.
FIG. 20 shows imaging of bone repair in clinical trial (extraction socket filling) using the composition in Example 3.

## DESCRIPTION OF EMBODIMENTS

In order to understand the technical features, objectives and beneficial effects of the present disclosure more clearly, the technical solutions of the present disclosure are described in detail as below, but they cannot be construed as limitations on the implementable scope of the present disclosure.

It should be noted that certain words are used in the description and claims to refer to particular components. It should be understood by those skilled in the art that the same component may be referred to by different terms. The present description and claims do not distinguish components in terms of differences in terms, but in terms of functional differences of components. As used throughout the description and claims, the terms "comprise" or "include" are open-ended terms and should be construed as "including but not limited to". The following description illustrates preferred embodiments of the present disclosure, which are provided for the purpose of exemplifying the general principles of the description and are not intended to limit the scope of the present disclosure. The scope of protection of the present disclosure shall be defined in the appended claims.

The amino acid sequence of the recombinant human collagen used in the following examples is:

GPPGEPGNPGKPGSPGPAGSNGEPGPAGSPGEKGSQGSNGNPGPAGNQGQPGNKGSPGN PGKPGEPGSNGPQGEPGSQGNPGKNGQPGSPGSQGSPGNQGQPGKPGQPGEQGSPGNQ GPAGNEGPKGQPGQNGKPGSPGPPGEPGNPGKPGSPGPAGSNGEPGPAGSPGEKGSQGS NGNPGPAGNQGQPGNKGSPGNPGKPGEPGSNGPQGEPGSQGNPGKNGQPGSPGSQGSP GNQGQPGKPGQPGEQGSPGNQGPAGNEGPKGQPGQNGKPGTPGPPGEPGNPGKPGSPG PAGSNGEPGPAGSPGEKGSQGSNGNPGPAGNQGQPGNKGSPGNPGKPGEPGSNGPQGE PGSQGNPGKNGQPGSPGSQGSPGNQGQPGKPGQPGEQGSPGNQGPAGNEGPKGQPGQN GKP (SEQ ID No: 1).

The recombinant human collagen freeze-dried powder used in the following examples is obtained by optimized amino acid sequence design, high expression of novel recombinant human collagen in *Pichia pastoris,* large-scale fermentation and purification. Specifically: the hydrophilic Gly-X-Y repeat sequence, which is the minimal repeating unit of human type I collagen, is subjected to targeted arrangement and combination to design a novel collagen nucleotide sequence, and then expressed in *Pichia pastoris* host strain GS115 via electroporation using a *Pichia pastoris* expression vector pPIC9K; after antibiotic G418 screening, the expression of the high-copy strains is amplified through fermentation; and purification is performed by ultrafiltration and ion exchange chromatography to obtain the high-purity recombinant human collagen.

The "nanoflower"-structured coralline hydroxyapatite used in the following examples is prepared by soaking coral stone in a cutting protective agent, i.e., 30% propylene glycol, for 16 h, performing crushing and granulating to yield a coral sample, and then subjecting the coral sample to hydrothermal exchange. The step of the hydrothermal exchange comprises: immersing the coral sample with a saturated diammonium hydrogen phosphate solution, and performing a reaction at 0.1-3 Mpa and 150°C-220°C for 6-19 h.

Example 1

This example provided a composition comprising a self-assembled collagen and "nanoflower"-structured coralline hydroxyapatite, which was prepared by the following steps.

10 g freeze-dried powder of recombinant human collagen (the recombinant human collagen was a protein having the amino acid sequence of SEQ ID No: 1 in the sequence listing) was weighed, and uniformly suspended after adding 20 g of phosphate buffer (pH = 6.6), to prepare 30 g of a recombinant human collagen solution. 70 g of "nanoflower"-structured coralline hydroxyapatite, having a particle size of 0.25 mm-1 mm, a pore size of 100 μm-800 μm, a porosity of 50%-80%, and a conversion rate of 15%, was weighed and added to the above recombinant human collagen solution, and the resulting mixture was uniformly suspended to obtain 100 g of suspension. The suspension was added into a mold with φ 10 mm and a height of 15 mm, the mold was placed in a refrigerator at -80°C to allow rapid freezing for 60 min, then vacuum freeze-drying was performed, and 80 samples were prepared. The samples were placed into a nitrogen-filled vacuum oven with a vacuum degree set at -10 KPa, and self-assembly was allowed to proceed at 100°C for 6 h. After the self-assembly was completed, the samples were sealed in aluminum foil bags and then subjected to electron beam irradiation sterilization at an irradiation dose of 25 kGy to obtain the composition, with a porosity of 98.17%.

The freeze-drying in the above process was as follows:

a pre-freezing stage was carried out, wherein the temperature reached -50°C within 120 min and was then maintained for 180 min;

the pre-frozen product was subjected to sublimation under a vacuum degree set at 0.01 mbar, wherein the temperature reached -10°C within 60 min and was then maintained for 600 min;

the sublimated product was subjected to vacuum drying under a vacuum degree set at 0.1 mbar, wherein the temperature reached 25°C within 40 min and was then maintained for 240 min.

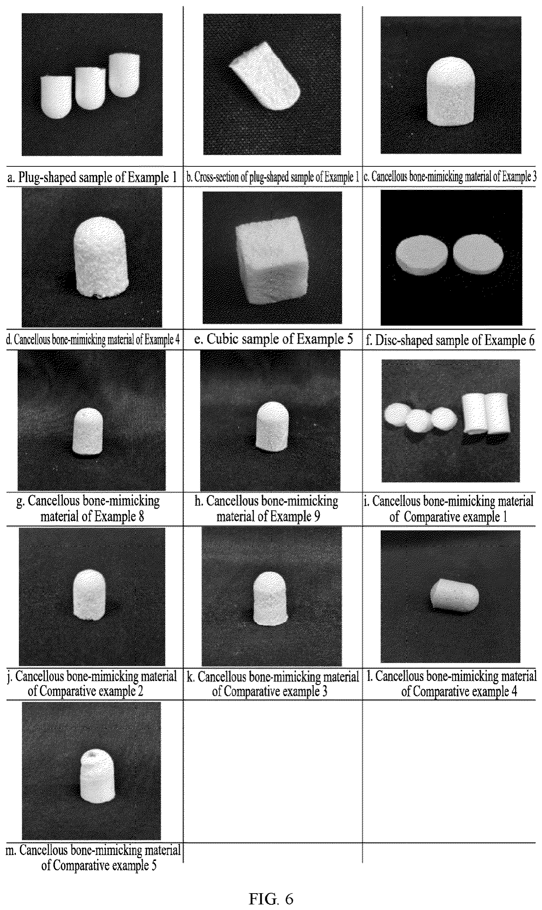

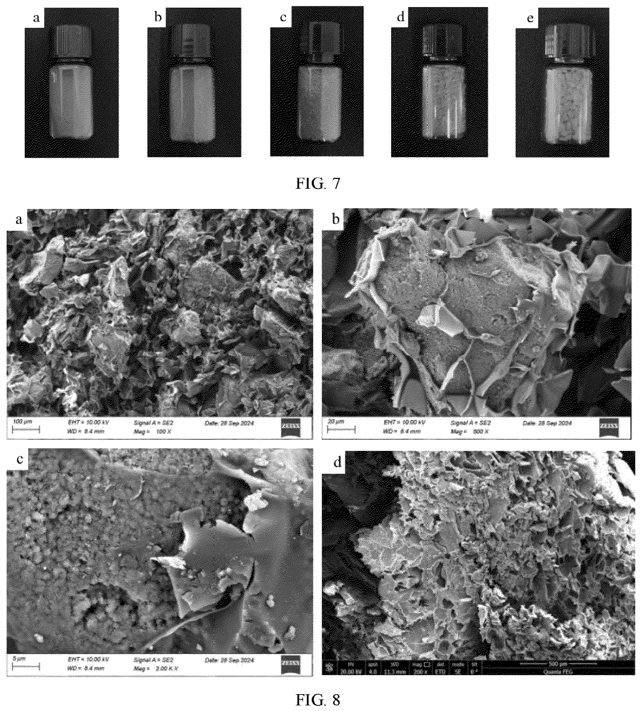

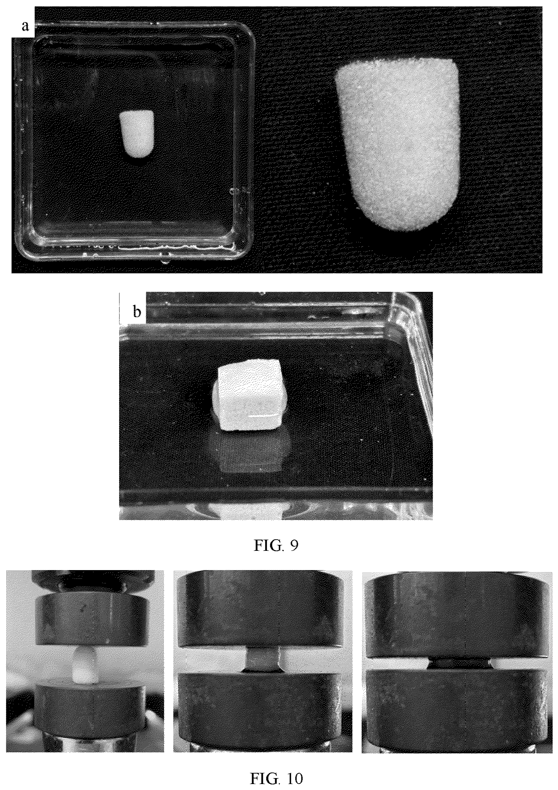

The appearance of the composition was as shown in FIG. 6a. The cross-sectional view as shown in FIG. 6b showed the uniform distribution of the "nanoflower"-structured coralline hydroxyapatite within the collagen scaffold visible to the naked eye. The scanning electron microscope images were as shown in FIGs. 8a, 8b, and 8c, indicating that the freeze-dried composition had a three-dimensional structure, in which the "nanoflower"-structured coralline hydroxyapatite was encapsulated by collagen and uniformly distributed in the collagen scaffold. The composition exhibited rapid rehydration, structural integrity without disintegration, and slight swelling (as shown in FIG. 9a), had good toughness

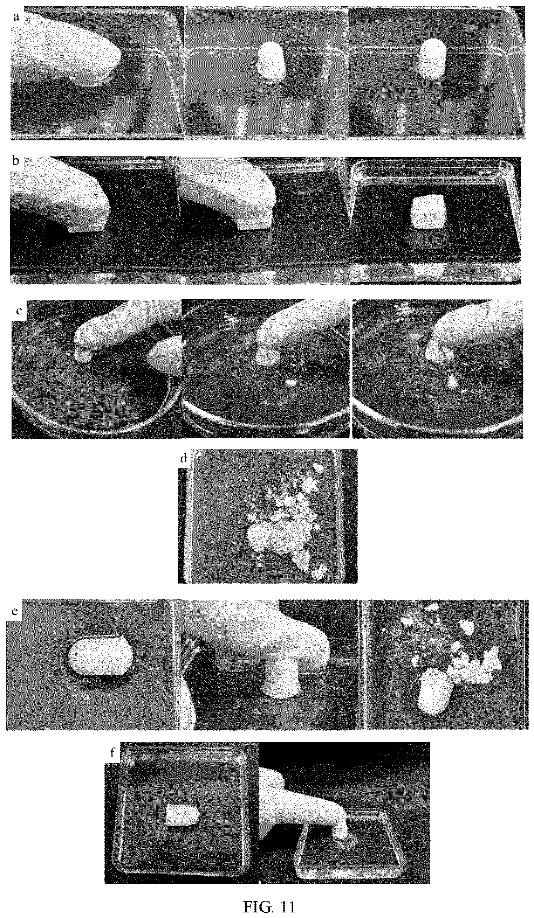

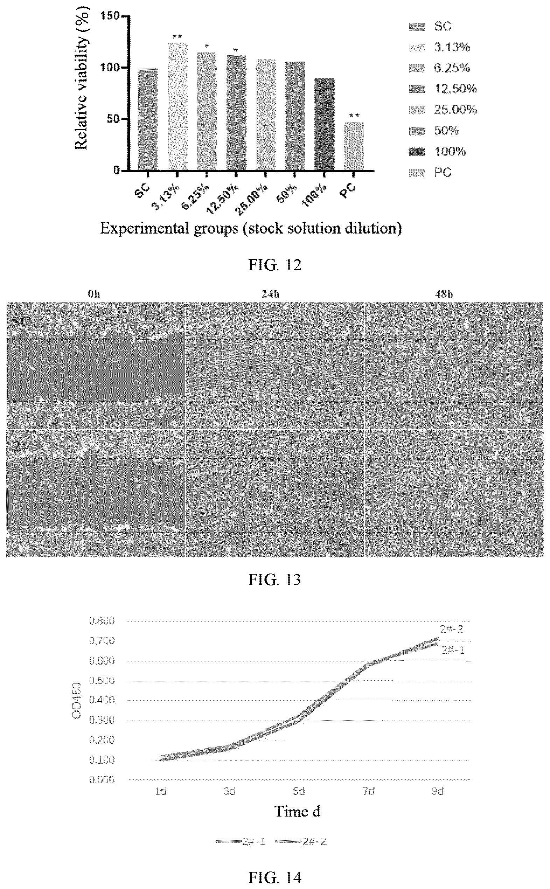

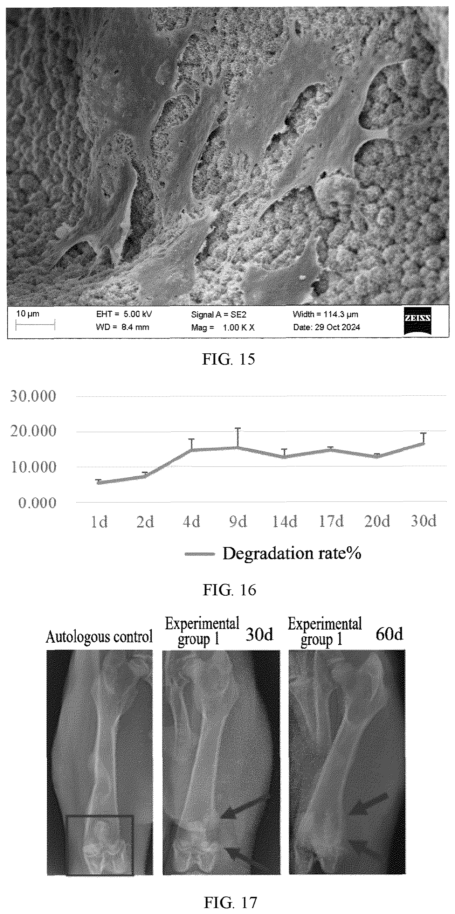

(as shown in FIG. 10), hydrophilic properties and shape memory function (as shown in FIG. 11a), and also demonstrated good cell compatibility (as shown in FIGs. 12, 13, 14, and 15) and biodegradability (as shown in FIG. 16).

(1) Bone repair experiment in rabbit lateral femoral condyle defect was performed using the composition.

Specifically,
Experimental animal: Healthy male New Zealand white rabbits aged 6 to 8 months and weighing 2.0 to 2.5 kg were selected.

Anesthesia and surgical preparation: All animals were fasted and deprived of water for 24 hours prior to surgery. The rabbits were anesthetized by intravenous injection of 2.5% pentobarbital sodium via marginal ear vein at a dose of 1 ml/kg. After successful anesthesia, the rabbit hind leg knee joint was slightly flexed, the skin was tightened, and the hair was removed.

Establishment of lateral femoral condyle defect model: A cranial drill with a diameter of 6 mm was used to vertically and intermittently drill into the lateral distal femur of the rabbit to form a bone defect with a diameter of 6 mm and depth of 5 mm. This process was performed under dripping of physiological saline.

Material implantation: The composition of Example 1 (φ 10 mm and a height of 15 mm) was trimmed to an appropriate size to obtain the bone repair material. Implantation was performed in the experimental groups according to Table 2, wherein each two groups shared one rabbit, and each two groups had 5 rabbits at each time point, for a total of three time points (30 days, 60 days, and 90 days).

Implantation was performed in the control group using a similar bone filling material (Geistlich Bio-Oss Collagen).

No material was implanted in the blank control group.

Table 2

| Experimental group / Observation time | Experiment group 1 | Control group (similar product) | Experiment group 2 | Blank control group |
|---|---|---|---|---|
| 30 days | 5 rabbits | | 5 rabbits | |
| 60 days | 5 rabbits | | 5 rabbits | |
| 90 days | 5 rabbits | | 5 rabbits | |
| Standby | 2 rabbits | | 2 rabbits | |

After defect closure, the lower limbs were not fixed, allowing the experimental rabbits to move freely. Penicillin was intramuscularly injected at 400000 U/d for 3 consecutive days after surgery, and the rabbits were housed separately in separate cages.

Experimental period:

The animals were sacrificed at 30, 60, and 90 days, respectively.

General observation:

The postoperative diet, activity, and condition of the skin and soft tissue around the incision were observed, including the healing of the incision and symptoms of infection and inflammation. The surface morphology of the specimens was observed for deformities, etc.

X-ray examination:

The anteroposterior and lateral X-ray examination of the bilateral distal femur was performed at 30, 60, and 90 days, respectively (imaging conditions: 70 KV, 80 mA, and 32 ms) to observe the repair of bone defects and the degradation of the material.

MicroCT examination:

The animals were sacrificed at 30, 60, and 90 days, respectively, fixed in 10% formaldehyde for 1 week, and then

washed and subjected to MicroCT examination.

The growth of new bone and the changes in the bone defect area were qualitatively analyzed through the three-dimensional reconstruction images from MicroCT.

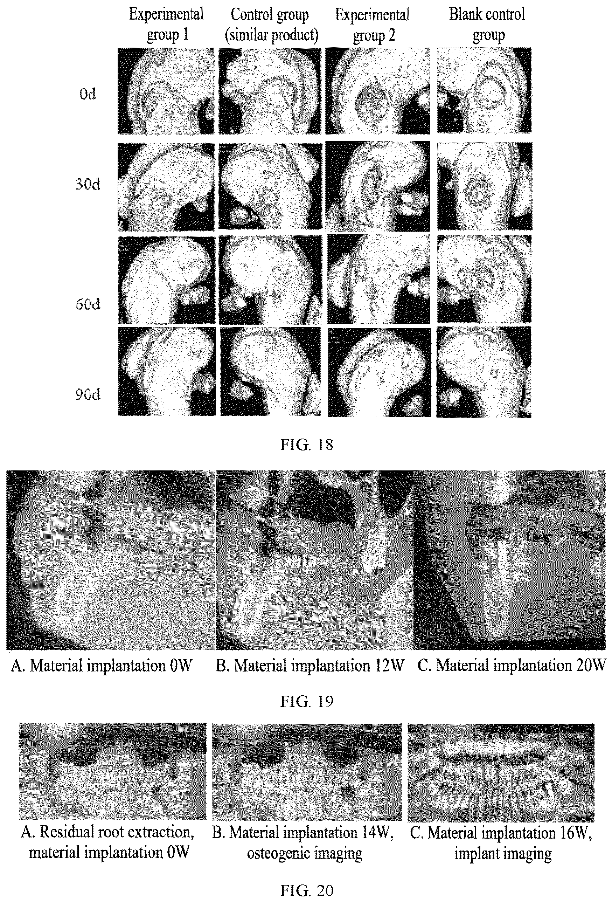

The results demonstrated that the material had a good bone repair effect, with the rate of bone repair superior to that of the similar bone filling material (Geistlich Bio-Oss Collagen) (as shown in FIG. 17 and FIG. 18).

(2) Clinical trial (extraction socket filling experiment) was performed using the composition. Specifically:

Patient selection and preoperative preparation:

Suitable patients were selected for the extraction socket filling experiment, excluding those with surgical contraindications such as diabetes and osteoporosis. Patients were required to sign informed consent forms.

Minimally invasive tooth extraction:

Minimally invasive tooth extraction was performed under local anesthesia to minimize trauma to the alveolar bone and soft tissue. The extraction socket was ensured to retain at least three bone walls.

Selection and application of bone material:

The composition of Example 1 (φ 10 mm and a height of 15 mm) was trimmed to an appropriate size to obtain the bone repair material. On the basis of its rapid blood absorption property, the material was directly packed into the extraction socket without pre-rehydration, followed by direct wound closure using suture without collagen membrane coverage.

Postoperative treatment:

The healing of soft and hard tissues was observed after surgery, and imaging examinations (such as cone-beam CT or X-ray) were performed 3-6 months after surgery to evaluate changes in bone volume.

Experimental results and analysis:

The experimental results showed that (as shown in FIG. 19) the material could perfectly repair an approximately 11 mm L-shaped bone defect, and no membrane coverage or metallic fixation was required during surgery. Osteogenesis occurred at 3 months, dental implantation could be performed at 5 months, complete integration into autologous bone was achieved, no foreign body or bone collapse was observed, and the height and width of the alveolar ridge were maintained.

Example 2

This example provided a composition comprising a self-assembled collagen and "nanoflower"-structured coralline hydroxyapatite, which was prepared by the following steps.

20 g freeze-dried powder of recombinant human collagen (the recombinant human collagen was the protein having the amino acid sequence of SEQ ID No: 1 in the sequence listing) was weighed, and uniformly suspended after adding 25 g of phosphate buffer (pH = 6.6) to prepare 45 g of recombinant human collagen solution. 55 g of the "nanoflower"-structured coralline hydroxyapatite, having a particle size of 0.25 mm-1 mm, a pore size of 100 μm-800 μm, a porosity of 50%-80%, and a conversion rate of 20%, was weighed and added to the above recombinant human collagen solution, and the resulting mixture was uniformly suspended to obtain 100 g of suspension. The suspension was added into a mold with φ 10 mm and a height of 15 mm, the mold was placed in a refrigerator at -80°C to allow rapid freezing for 100 min, then vacuum freeze-drying was performed, and 80 block samples were prepared. Self-assembly was performed at 150°C for 8 h in an electric-heating vacuum drying oven with a vacuum degree set at -0.095 MPa. The block samples were then crushed using a pulverizer (JC-FW-100), followed by sieving through a 10 mesh-65 mesh sieve. The sieved samples were loaded into vials, sealed in aluminum foil bags, and then subjected to electron beam irradiation sterilization at an irradiation dose of 25 kGy to obtain the composition, with a porosity of 98.62%.

The freeze-drying in the above process was as follows:

a pre-freezing stage was performed, wherein the temperature reached -45°C within 100 min and was then maintained for 200 min;

the pre-frozen product was subjected to sublimation under a vacuum degree set at 0.01 mbar, where the temperature reached -10°C within 60 min and was then maintained for 600 min;

the sublimated product was subjected to vacuum drying under a vacuum degree set at 0.08 mbar, where the

temperature reached 27°C within 50 min and was then maintained for 250 min.

The appearance of the composition (granular) was as shown in FIG. 7, where FIGs. 7a-7e showed samples having particle size distribution ranges of < 0.25 mm, 0.25-0.3 mm, 0.3-0.5 mm, 0.5-1 mm, and 1-2 mm, respectively.

**Example 3**

This example provided a composition comprising a self-assembled collagen and "nanoflower"-structured coralline hydroxyapatite, which was prepared by the following steps.

15 g freeze-dried powder of recombinant human collagen (the recombinant human collagen was the protein having the amino acid sequence of SEQ ID No: 1 in the sequence listing) was weighed, and uniformly suspended after adding 45 g of water for injection to prepare 60 g of recombinant human collagen solution. 40 g of the "nanoflower"-structured coralline hydroxyapatite, having a particle size of 0.5 mm-1 mm, a pore size of 100 μm-800 μm, a porosity of 50%-80%, and a conversion rate of 22%, was weighed and added to the above recombinant human collagen solution, and the resulting mixture was uniformly suspended to obtain 100 g of suspension. The suspension was added into a mold with φ 10 mm and a height of 15 mm, the mold was placed in a refrigerator at -80°C to allow rapid freezing for 120 min, then vacuum freeze-drying was performed, and 80 block samples were prepared. Self-assembly was performed at 170°C for 7 h in an electric-heating vacuum drying oven with a vacuum degree set at -0.095 MPa. After the self-assembly was completed, the samples were sealed in double-layer blister packs and then subjected to ethylene oxide sterilization (sterilization parameters: sterilization temperature: 55°C, sterilization humidity: 50%, ethylene oxide concentration: 630 $g/m^3$, vacuum degree: -15 KPa, sterilization time: 10 h) to obtain the composition, with a porosity of 96.58%.

The freeze-drying in the above process was as follows:

a pre-freezing stage was performed, wherein the temperature reached -50°C within 140 min and was then maintained for 190 min;

the pre-frozen product was subjected to sublimation under a vacuum degree set at 0.01 mbar, where the temperature reached -10°C within 60 min and was then maintained for 600 min;

the sublimated product was subjected to vacuum drying under a vacuum degree set at 0.06 mbar, where the temperature reached 20°C within 30 min and was then maintained for 250 min.

The appearance of the composition was as shown in FIG. 6c.

Clinical trial (extraction socket filling experiment) was performed using the composition. Specifically:

Patient selection and preoperative preparation: Suitable patients were selected for the extraction socket filling experiment, excluding those with surgical contraindications such as diabetes and osteoporosis. Patients were required to sign informed consent forms.

Minimally invasive tooth extraction: Minimally invasive tooth extraction was performed under local anesthesia to minimize trauma to the alveolar bone and soft tissue. The extraction socket was ensured to retain at least three bone walls.

Selection and application of bone material: The composition of Example 3 (φ 10 mm and a height of 15 mm) was trimmed to an appropriate size to obtain the bone repair material. On the basis of its rapid blood absorption property, the material was directly packed into the extraction socket without pre-rehydration, followed by direct wound closure using suture without collagen membrane coverage.

Postoperative treatment: The healing of soft and hard tissues was observed after surgery, and imaging examinations (such as cone-beam CT or X-ray) were performed 3-6 months after surgery to evaluate changes in bone volume.

Experimental results and analysis: The experimental results showed that (as shown in FIG. 20) the material could perfectly repair bone defects within 14 weeks, no membrane coverage or metallic fixation was required during surgery, and the height and width of the alveolar ridge could be maintained. Upon examination, osteogenesis was observed at 3 months, and the patient underwent dental implantation surgery at the 2nd week.

**Example 4**

This example provided a composition comprising a self-assembled collagen and "nanoflower"-structured coralline hydroxyapatite, which was prepared by the following steps.

10 g freeze-dried powder of recombinant human collagen (the recombinant human collagen was the protein having the amino acid sequence of SEQ ID No: 1 in the sequence listing) was weighed, and uniformly suspended after adding 40 g of water for injection to prepare 50 g of recombinant human collagen solution. 50 g of the "nanoflower"-structured coralline hydroxyapatite, having a particle size of 0.5 mm-1.25 mm, a pore size of 100 μm-800 μm, a porosity of 50%-80%, and a conversion rate of 18%, was weighed and added to the above recombinant human collagen solution, and

the resulting mixture was uniformly suspended to obtain 100 g of suspension. The suspension was added into a mold with φ 10 mm and a height of 15 mm, the mold was placed in a refrigerator at -80°C to allow rapid freezing for 180 min, then vacuum freeze-drying was performed, and 80 block samples were prepared. Self-assembly was performed at 180°C for 7 h in a nitrogen-filled vacuum oven with a vacuum degree set at -15 KPa. After the self-assembly was completed, the samples were sealed in aluminum foil bags and then subjected to cobalt 60 irradiation sterilization at an irradiation dose of 25 kGy to obtain the composition, with a porosity of 96.23%.

The freeze-drying in the above process was as follows:

a pre-freezing stage was performed, wherein the temperature reached -50°C within 160 min and was then maintained for 220 min;
the pre-frozen product was subjected to sublimation under a vacuum degree set at 0.05 mbar, where the temperature reached -10°C within 50 min and was then maintained for 450 min;
the sublimated product was subjected to vacuum drying under a vacuum degree set at 0.07 mbar, where the temperature reached 30°C within 40 min and was then maintained for 220 min.

The appearance of the composition was as shown in FIG. 6d.

**Example 5**

This example provided a composition comprising a self-assembled collagen and "nanoflower"-structured coralline hydroxyapatite, which was prepared by the following steps.

10 g freeze-dried powder of recombinant human collagen (the recombinant human collagen was the protein having the amino acid sequence of SEQ ID No: 1 in the sequence listing) was weighed, and uniformly suspended after adding 40 g of purified water to prepare 50 g of recombinant human collagen solution. 50 g of the "nanoflower"-structured coralline hydroxyapatite, having a particle size of 0.5 mm-1 mm, a pore size of 100 μm-800 μm, a porosity of 50%-80%, and a conversion rate of 15%, was weighed and added to the above recombinant human collagen solution, and the resulting mixture was uniformly suspended to obtain 100 g of suspension. The suspension was added into a 1 cm × 1 cm × 1 cm mold, the mold was placed in a refrigerator at -80°C to allow rapid freezing for 180 min, then vacuum freeze-drying was performed, and 80 block samples were prepared. Self-assembly was performed at 180°C for 6 h in an electric-heating vacuum drying oven with a vacuum degree set at -0.095 MPa. After the self-assembly was completed, the samples were sealed in aluminum foil bags and then subjected to electron beam irradiation sterilization at an irradiation dose of 25 kGy to obtain the composition, with a porosity of 96.06%.

The freeze-drying in the above process was as follows:

a pre-freezing stage was performed, wherein the temperature reached -50°C within 200 min and was then maintained for 200 min;
the pre-frozen product was subjected to sublimation under a vacuum degree set at 0.1 mbar, where the temperature reached -10°C within 60 min and was then maintained for 500 min;
the sublimated product was subjected to vacuum drying under a vacuum degree set at 0.08 mbar, where the temperature reached 25°C within 50 min and was then maintained for 200 min.

The appearance of the composition was as shown in FIG. 6e. The composition exhibited structural integrity without disintegration upon rehydration (as shown in FIG. 9b), and had good toughness, hydrophilic properties, and shape memory function (as shown in FIG. 11b).

**Example 6**

This example provided a composition comprising a self-assembled collagen and "nanoflower"-structured coralline hydroxyapatite, which was prepared by the following steps.

12 g freeze-dried powder of recombinant human collagen (the recombinant human collagen was the protein having the amino acid sequence of SEQ ID No: 1 in the sequence listing) was weighed, and uniformly suspended after adding 40 g of purified water to prepare 52 g of recombinant human collagen solution. 48 g of the "nanoflower"-structured coralline hydroxyapatite, having a particle size of 0.25 mm-1 mm, a pore size of 100 μm-800 μm, a porosity of 50%-80%, and a conversion rate of 15%, was weighed and added to the above recombinant human collagen solution, and the resulting mixture was uniformly suspended to obtain 100 g of suspension. The suspension was added into a mold with φ 30 mm and a height of 5 mm, the mold was placed in a refrigerator at -80°C to allow rapid freezing for 180 min, and then vacuum freeze-drying was performed, and 26 sheet samples were prepared. Self-assembly was performed at 220°C for 6 h in an electric-heating vacuum drying oven with a vacuum degree set at -0.095 MPa. After the self-assembly was

completed, the samples were sealed in aluminum foil bags and then subjected to electron beam irradiation sterilization at an irradiation dose of 25 kGy to obtain the composition, with a porosity of 97.79%.

The freeze-drying in the above process was as follows:

a pre-freezing stage was performed, wherein the temperature reached -50°C within 200 min and was then maintained for 200 min;
the pre-frozen product was subjected to sublimation under a vacuum degree set at 0.1 mbar, where the temperature reached -10°C within 60 min and was then maintained for 500 min;
the sublimated product was subjected to vacuum drying under a vacuum degree set at 0.08 mbar, where the temperature reached 25°C within 50 min and was then maintained for 200 min.

The appearance of the composition was as shown in FIG. 6f.

**Example 7**

This example provided a composition comprising a self-assembled collagen and "nanoflower"-structured coralline hydroxyapatite, which was prepared by the following steps.

Three 15 g portions of recombinant human collagen freeze-dried powder (the recombinant human collagen was the protein having the amino acid sequence of SEQ ID No: 1 in the sequence listing) were weighed separately. Each portion was uniformly suspended after adding 40 g of purified water to prepare three 55 g portions of recombinant human collagen solution. Three types of 45 g of the "nanoflower"-structured coral (with a conversion rate of 10%, 55% and 80%, respectively), having a particle size of 0.5 mm-1 mm, a pore size of 100 μm-800 μm, and a porosity of 50%-80%, were weighed separately. Each type was added to the above recombinant human collagen solution, and the resulting mixture was uniformly suspended to obtain three 100 g portions of suspension. The suspension was added into a mold with φ 10 mm and a height of 15 mm, the mold was placed in a refrigerator at -80°C to allow rapid freezing for 200 min, and then vacuum freeze-drying was performed. The dried samples were placed into an electric-heating vacuum drying oven with a vacuum degree set at -0.095 MPa, and self-assembly was performed at 180°C for 6 h. After the self-assembly was completed, the samples were sealed in aluminum foil bags and then subjected to electron beam irradiation sterilization at an irradiation dose of 25 kGy to obtain the composition.

The freeze-drying in the above process was as follows:

a pre-freezing stage was performed, wherein the temperature reached -50°C within 200 min and was then maintained for 200 min;
the pre-frozen product was subjected to sublimation under a vacuum degree set at 0.1 mbar, where the temperature reached -10°C within 60 min and was then maintained for 500 min;
the sublimated product was subjected to vacuum drying under a vacuum degree set at 0.08 mbar, where the temperature reached 25°C within 50 min and was then maintained for 200 min.

The pore size structure of the composition was as shown in Table 3. As can be seen from Table 3, the higher the conversion rate of the "nanoflower"-structured coralline hydroxyapatite, the larger the pore size and the greater the porosity, resulting in a composition with higher porosity.

Table 3

| Conversion rate of "nanoflower"-structured coralline hydroxyapatite | Pore size distribution range of "nanoflower"-structured coralline hydroxyapatite | Porosity of "nanoflower"-structured coralline hydroxyapatite | Porosity of composition |
|---|---|---|---|
| 10% | 143 μm-365 μm; | 66.41%-86.95% | 85.33%-98.69% |
| 55% | 336 μm-510 μm; | 74.91%-84.35% | 93.76%-98.22% |
| 80% | 636 μm-800 μm; | 82.91%-88.35% | 96.85%-99.09% |

**Example 8**

This example provided a composition comprising a self-assembled collagen and "nanoflower"-structured coralline hydroxyapatite, which was prepared by the following steps.

5 g freeze-dried powder of recombinant human collagen (the recombinant human collagen was the protein having the amino acid sequence of SEQ ID No: 1 in the sequence listing) was weighed, and uniformly suspended after

adding 20 g of phosphate buffer (pH = 6.6) to prepare 25 g of recombinant human collagen solution. 5 g of cross-linked porous starch was weighed and added to the prepared recombinant human collagen solution, and the resulting mixture was uniformly mixed. 70 g of the "nanoflower"-structured coralline hydroxyapatite, having a particle size of 0.25 mm-1 mm, a pore size of 100 μm-800 μm, a porosity of 50%-80%, and a conversion rate of 15%, was weighed and added to the above mixed solution, and the resulting mixture was uniformly mixed to obtain 100 g of a viscous solution. The suspension was added into a mold with φ 10 mm and a height of 15 mm, the mold was placed in a refrigerator at -80°C for rapid freezing for 200 min, and then vacuum freeze-drying was performed to prepare 80 samples. The samples were placed into a nitrogen-filled vacuum oven with a vacuum degree set at -10 KPa, and self-assembly was performed at 100°C for 6 h. After the self-assembly was completed, the samples were sealed in aluminum foil bags and then subjected to electron beam irradiation sterilization at an irradiation dose of 25 kGy to obtain the composition, with a porosity of 97.43%.

The freeze-drying in the above process was as follows:

a pre-freezing stage was performed, wherein the temperature reached -50°C within 120 min and was then maintained for 180 min;

the pre-frozen product was subjected to sublimation under a vacuum degree set at 0.01 mbar, where the temperature reached -10°C within 60 min and was then maintained for 600 min;

the sublimated product was subjected to vacuum drying under a vacuum degree set at 0.1 mbar, where the temperature reached 25°C within 40 min and was then maintained for 240 min.

The appearance of the composition was as shown in FIG. 6g.

**Example 9**

This example provided a composition comprising a self-assembled collagen and "nanoflower"-structured coralline hydroxyapatite, which was prepared by the following steps.

5 g freeze-dried powder of recombinant human collagen (the recombinant human collagen was the protein having the amino acid sequence of SEQ ID No: 1 in the sequence listing) was weighed, and uniformly suspended after adding 20 g of phosphate buffer (pH = 6.6) to prepare 25 g of recombinant human collagen solution. 5 g of sodium carboxymethyl cellulose was weighed and added to the prepared recombinant human collagen solution, and the resulting mixture was uniformly mixed. 70 g of the "nanoflower"-structured coralline hydroxyapatite, having a particle size of 0.25 mm-1 mm, a pore size of 100 μm-800 μm, a porosity of 50%-80%, and a conversion rate of 10%, was weighed and added to the above mixed solution, and the resulting mixture was uniformly mixed to obtain 100 g of a viscous solution. The suspension was added into a mold with φ 10 mm and a height of 15 mm, the mold was placed in a refrigerator at -80°C for rapid freezing for 200 min, and then vacuum freeze-drying was performed to prepare 80 samples. The samples were placed into a nitrogen-filled vacuum oven with a vacuum degree set at -10 KPa, and self-assembly was performed at 100°C for 6 h. After the self-assembly was completed, the samples were sealed in aluminum foil bags and then subjected to electron beam irradiation sterilization at an irradiation dose of 25 kGy to obtain the composition, with a porosity of 98.83%.

The freeze-drying in the above process was as follows:

a pre-freezing stage was performed, wherein the temperature reached -50°C within 120 min and was then maintained for 180 min;

the pre-frozen product was subjected to sublimation under a vacuum degree set at 0.01 mbar, where the temperature reached -10°C within 60 min and was then maintained for 600 min;

the sublimated product was subjected to vacuum drying under a vacuum degree set at 0.1 mbar, where the temperature reached 25°C within 40 min and was then maintained for 240 min.

The appearance of the composition was as shown in FIG. 6h.

**Comparative example 1**

This comparative example provided a composition prepared by the following steps.

12 g freeze-dried powder of recombinant human collagen (the recombinant human collagen was the protein having the amino acid sequence of SEQ ID No: 1 in the sequence listing) was weighed, and uniformly suspended after adding 40 g of purified water to prepare 52 g of recombinant human collagen solution. 48 g of nano-hydroxyapatite (a nano-scale material without pore structure; manufacturer: NANJING EMPEROR NANO MATERIAL CO., LTD; particle size: 20 nm; purity: 99%) was weighed and added to the above recombinant human collagen solution, and the resulting mixture was uniformly suspended to obtain 100 g of suspension. The suspension was added into a mold with φ 10 mm and a height of 15 mm, the mold was placed in a refrigerator at -80°C to allow rapid freezing for 180 min, then vacuum freeze-drying was

performed, and 80 block samples were prepared. Self-assembly was performed at 180°C for 6 h in an electric-heating vacuum drying oven with a vacuum degree set at -0.095 MPa to obtain the composition.

The freeze-drying in the above process was as follows:

a pre-freezing stage was performed, wherein the temperature reached -50°C within 200 min and was then maintained for 200 min;
the pre-frozen product was subjected to sublimation under a vacuum degree set at 0.1 mbar, where the temperature reached -10°C within 60 min and was then maintained for 500 min;
the sublimated product was subjected to vacuum drying under a vacuum degree set at 0.08 mbar, where the temperature reached 25°C within 50 min and was then maintained for 200 min.

The appearance of the composition was as shown in FIG. 6i, and the scanning electron microscope image was as shown in FIG. 8d, indicating that the freeze-dried nano-hydroxyapatite was encapsulated by collagen, forming a relatively dense three-dimensional structure. The composition was used to prepare a bone repair material for the bone repair experiment in the rabbit lateral femoral condyle defect (as shown in experimental group 2 in FIG. 18). The results demonstrated that the bone repair effect of this material was superior to that of the blank control but inferior to that of Example 1. The porous structure of the "nanoflower"-structured coralline hydroxyapatite of the present disclosure more closely resembled that of human cancellous bone, and the three-dimensional porous channels formed by the porous structure increased the interface between the material and the tissue in the implantation area, facilitating an accelerated interfacial binding reaction process and providing space for osteoinductive substances *in vivo.* Furthermore, the interconnected pores facilitated nutrient transport and the interconnection of fibrous and vascular tissues, which was more conducive to guiding the ingrowth of new bone. Meanwhile, the conversion rate of the "nanoflower"-structured coralline hydroxyapatite was appropriate, enabling the degradation along with bone repair *in vivo,* while the nano-hydroxyapatite was degraded more slowly, requiring a longer period to be completely absorbed and replaced *in vivo.*

## Comparative example 2

This comparative example provided a composition prepared by the following steps.

1 g of collagen sponge (generic name: medical collagen sponge, trade name: Kejibang, and manufacturer: WUXI BIOT ENGINEERING CO., LTD.) was weighed, cut into pieces, and dispersed in 50 ml of purified water, and homogenization treatment was then performed using a homogenizer for 10 minutes to obtain a collagen slurry. 8 g of the "nanoflower"-structured coralline hydroxyapatite particles, having a particle size of 0.25 mm-1 mm, a pore size of 100 μm-800 μm, a porosity of 50-80%, and a conversion rate of 15%, was mixed with the collagen slurry, and the resulting mixture was mixed and stirred using a magnetic stirrer for 20 minutes to obtain a collagen-coralline hydroxyapatite mixed slurry. The resulting collagen-coralline hydroxyapatite mixed slurry was transferred into a mold and subjected to compression dewatering for 12 hours. After demolding, freeze-drying was performed to obtain a molded collagen-coralline hydroxyapatite scaffold. The above collagen-coralline hydroxyapatite scaffold was placed in an electric-heating vacuum drying oven, and heated to 60°C-180°C under a vacuum degree set at -0.095 MPa for thermal cross-linking treatment for 2 hours to obtain the final collagen-coralline hydroxyapatite composite scaffold, with a porosity of 88.05%.

The freeze-drying in the above process was as follows:

a pre-freezing stage was performed, wherein the temperature reached -50°C within 200 min and was then maintained for 200 min;
the pre-frozen product was subjected to sublimation under a vacuum degree set at 0.1 mbar, where the temperature reached -10°C within 60 min and was then maintained for 500 min;
the sublimated product was subjected to vacuum drying under a vacuum degree set at 0.08 mbar, where the temperature reached 25°C within 50 min and was then maintained for 200 min.

The appearance of the collagen-coralline hydroxyapatite composite scaffold was as shown in FIG. 6j, and the rehydration experiment was as shown in FIG. 11c, indicating that the collagen-coralline hydroxyapatite composite scaffold exhibited disintegration upon rehydration and was easily crushed by hand, and that the strength and toughness of the sample were significantly inferior to those of the product in the examples, that is, the product in the examples better met the requirements for clinical use.

## Comparative example 3

This comparative example provided a composition prepared by the following steps.

10 g freeze-dried powder of recombinant human collagen (raw material from other companies on the market,

white or off-white sponge-like solid with a purity of 99.9%) was weighed, and uniformly suspended after adding 20 g of phosphate buffer (pH = 6.6) to prepare 30 g of recombinant human collagen solution. 70 g of the "nanoflower"-structured coralline hydroxyapatite, having a particle size of 0.25 mm-1 mm, a pore size of 200 μm-800 μm, a porosity of 50-70%, and a conversion rate of 15%, was weighed and added to the above recombinant human collagen solution, and the resulting mixture was uniformly suspended to obtain 100 g of suspension. The suspension was added into a mold with φ 10 mm and a height of 15 mm, the mold was placed in a refrigerator at -80°C to allow rapid freezing for 60 min, then vacuum freeze-drying was performed, and 80 samples were prepared. The samples were placed into a nitrogen-filled vacuum oven with a vacuum degree set at -10 KPa, and treated at 100°C for 6 h to obtain the composition, with a porosity of 78.55%.

The freeze-drying in the above process was as follows:

(1) a pre-freezing stage was performed, wherein the temperature reached -50°C within 120 min and was then maintained for 180 min;
(2) the pre-frozen product was subjected to sublimation under a vacuum degree set at 0.01 mbar, where the temperature reached -10°C within 60 min and was then maintained for 600 min;
(3) the sublimated product was subjected to vacuum drying under a vacuum degree set at 0.1 mbar, where the temperature reached 25°C within 40 min and was then maintained for 240 min.

The appearance of the composition was as shown in FIG. 6k, exhibiting a slightly yellowish color. The rehydration experiment was as shown in FIG. 11d, indicating that the composition exhibited slight dissolution and particle disintegration upon rehydration, and that the strength and toughness of the sample were significantly inferior to those of the product in the examples, that is, the product in the examples better met the requirements for clinical use.

**Comparative example 4**

This comparative example provided a composition comprising a self-assembled collagen and "nanoflower"-structured coralline hydroxyapatite, which was prepared by the following steps.

6 g freeze-dried powder of recombinant human collagen (the recombinant human collagen was the protein having the amino acid sequence of SEQ ID No: 1 in the sequence listing) was weighed, and uniformly suspended after adding 14 g of purified water to prepare 20 g of recombinant human collagen solution. 80 g of the "nanoflower"-structured coralline hydroxyapatite, having a particle size of 0.25 mm-1 mm, a pore size of 200 μm-800 μm, a porosity of 50-70%, and a conversion rate of 15%, was weighed and added to the above recombinant human collagen solution, and the resulting mixture was uniformly suspended to obtain 100 g of suspension. The suspension was added into a mold with φ 10 mm and a height of 15 mm, the mold was placed in a refrigerator at -80°C to allow rapid freezing for 180 min, then vacuum freeze-drying was performed, and 80 samples were prepared. Self-assembly was performed at 160°C for 6 h in an electric-heating vacuum drying oven with a vacuum degree set at -0.095 MPa to obtain the composition, with a porosity of 71.52%.

The freeze-drying in the above process was as follows:

(1) a pre-freezing stage was performed, wherein the temperature reached -50°C within 200 min and was then maintained for 200 min;
(2) the pre-frozen product was subjected to sublimation under a vacuum degree set at 0.1 mbar, where the temperature reached -10°C within 60 min and was then maintained for 500 min;
(3) the sublimated product was subjected to vacuum drying under a vacuum degree set at 0.08 mbar, where the temperature reached 25°C within 50 min and was then maintained for 200 min.

The "nanoflower"-structured coralline hydroxyapatite in this composition accounted for 93% (dry weight). The appearance of the sample was as shown in FIG. 6l, exhibiting a slightly yellowish color. The rehydration experiment was as shown in FIG. 11e, indicating that the composition was relatively hard, and exhibited slow rehydration, and particle disintegration upon rehydration, and that the sample lacked toughness and was significantly inferior to those of the product in the examples, that is, the product in the examples better met the requirements for clinical use.

**Comparative example 5**

This comparative example provided a composition comprising a self-assembled collagen and "nanoflower"-structured coralline hydroxyapatite, which was prepared by the following steps.

10 g freeze-dried powder of recombinant human collagen (the recombinant human collagen was the protein having the amino acid sequence of SEQ ID No: 1 in the sequence listing) was weighed, and uniformly suspended after adding 20 g of phosphate buffer (pH = 6.6) to prepare 30 g of recombinant human collagen solution. 70 g of the "nanoflower"-structured coralline hydroxyapatite, having a particle size of 0.25 mm-1 mm, a pore size of 200 μm-800 μm, a

porosity of 50-70%, and a conversion rate of 15%, was weighed and added to the above recombinant human collagen solution, and the resulting mixture was uniformly suspended to obtain 100 g of suspension. The suspension was added into a mold with φ 10 mm and a height of 15 mm, the mold was placed in a refrigerator at -80°C to allow rapid freezing for 60 min, then vacuum freeze-drying was performed, and 80 samples were prepared. The samples were placed into a nitrogen-filled vacuum oven with a vacuum degree set at -10 KPa, and self-assembly was performed at 100°C for 6 h to obtain the composition, with a porosity of 78.18%.

The freeze-drying in the above process was as follows:

(1) a pre-freezing stage was performed, wherein the temperature reached -50°C within 60 min and was then maintained for 180 min;
(2) the pre-frozen product was subjected to sublimation under a vacuum degree set at 0.01 mbar, where the temperature reached -10°C within 300 min and was then maintained for 600 min;
(3) the sublimated product was subjected to vacuum drying under a vacuum degree set at 0.1 mbar, where the temperature reached 25°C within 40 min and was then maintained for 240 min.

This comparative example mainly involved slowing down the heating rate during the freeze-drying. The appearance of the composition sample was as shown in FIG. 6m. An excessively slow heating rate could lead to uneven temperature distribution within the product, resulting in structural collapse and reduced porosity of the sample. The rehydration experiment was as shown in FIG. 11f, indicating that the composition exhibited slow rehydration and particle disintegration upon rehydration, and that the sample lacked toughness, which was significantly inferior to those of the product in the examples, that is, the product in the examples better met the requirements for clinical use.

Taking the product obtained in Example 1 as a representative, the relevant performance tests were performed, specifically as follows:

**Appearance:** Visual observation under daylight lamp showed a yellow or pale yellow appearance with uniform color, as shown in FIGs. 6a and 6b.

**Compression deformation test:** The test was performed in accordance with ASTM F1566-15 "Standard Test Methods for Evaluation of In Vitro Properties of Resilient Materials Used as Medical Sponges". The material was rehydrated and then placed on the compression fixture platform of an electronic universal testing machine. The test speed was set at 10 mm/min to simulate the stress rate of the material in practical applications. The electronic universal testing machine was started to begin the compression test. The test process was monitored in real time, and the deformation during compression was recorded. The results were as shown in FIG. 10, indicating that the cancellous bone material exhibited good toughness and mechanical strength, being resistant to crushing or crumbling under pressure.

**Swelling property test:** The samples in each group were measured using a vernier caliper for the actual size V0, and then weighed to obtain the initial weight (w0), and the data were recorded. The samples were soaked in distilled water for 30 s and then taken out, and the surface moisture was absorbed with filter paper. The samples were weighed again (w), and the sizes after swelling V were measured using a vernier caliper. Swelling ratio (%) = ((w - wo)/wo) × 100%; volume ratio before and after swelling = V/V0. The results were as shown in Table 4, indicating that the material exhibited good water absorption, with swelling volume confined within a certain range, ensuring that it would not compress surrounding tissues during clinical use.

Table 4 Swelling test results

| | |
|---|---|
| Swelling ratio/% (*n* = 10) | 48.22±6.33 |
| Volume ratio before and after swelling (*n* = 10) | 1.48±0.06 |

**Cytotoxicity test:** The cytotoxicity experiment was performed on the composition obtained in Example 1 (two parallel experiments were performed, with sample 1 and sample 2 both being products obtained in Example 1) in accordance with GB/T 16886.5-2017 "Biological Evaluation of Medical Devices-Part 5: Tests for In Vitro Cytotoxicity (MTT Method)".The specific procedures were as follows: High-glucose DMEM culture medium was used as the extraction medium. The composition was first fully swollen and then extracted at a ratio of 0.1 g/ml. The extraction was performed at 37°C for 72 h. L929 cells ($1.2 \times 10^5$ cells/mL) in the logarithmic growth phase were seeded into 96-well plates at 100 μL/well, and cultured for 24 h. When the cell confluence in the 96-well plates reached 40% to 60%, the following treatments were administered: ① Sample group: 100 μL of culture medium containing different dilution concentrations (100%, 50%, 25%, 12.5%, 6.25%, and 3.13%) of extract stock solution was added to each well. ② Positive control group (PC): 100 uL of culture medium containing 5% DMSO was added. ③ Blank zeroing group (BC): No cells, only 100 μL of culture medium was added. ④ Blank control group (SC): Cells were present, and only 100 μL of culture medium was added. The cells were cultured for 24 h. The supernatant was discarded, the culture medium was replaced with a culture medium containing MTT

(0.5 mg/mL) at 150 μL/well, and the culture was continued for 4 h. The supernatant was discarded, and DMSO was added at 150 μL/well. After thorough oscillation and color development, the $OD_{490}$ was measured using a microplate reader. The relative cell viability was calculated according to Equation (1):

$$\text{Relative cell viability (\%)} = \left(\frac{\text{Sample well OD} - \text{Blank well OD}}{\text{Control well OD} - \text{Blank well OD}}\right) \times 100\% \quad \text{Equation (1).}$$

Cytotoxicity judgement criteria: If the relative cell viability was greater than 70%, it was considered that there is no cytotoxic reaction; otherwise, it was considered to be potentially cytotoxic.

The experimental results were as shown in Table 5 and FIG. 12.

Table 5 Cytotoxicity test results of composition-L929

| **Group** | **SC** | **3.13%** | **6.25%** | **12.50%** | **25.00%** | **50%** | **100%** | **PC** |
|---|---|---|---|---|---|---|---|---|
| mean | 3.955 | 1.189 | 1.099 | 1.07 | 1.038 | 1.012 | 0.856 | 0.452 |
| Relative viability% | 100.00% | 124.51% | 115.12% | 112.05% | 108.73% | 106.01% | 89.70% | 47.35% |
| SD | 0.07 | 0.03 | 0.03 | 0.02 | 0.01 | 0.02 | 0.04 | 0.08 |
| P | / | 0.01 | 0.03 | 0.05 | 0.11 | 0.24 | 0.11 | 0 |

It could be seen from Table 5 that the composition of the present disclosure exhibited good biocompatibility and no cytotoxicity.

**Cell migration:** Extraction: 4 g of the composition was added to 20 mL of physiological saline, and extraction was performed at 121°C for 1 hour in a. pressure steam sterilizer

Coating: 2 mL of the test sample extract was added to each well of 6-well plates, and incubation was performed in an incubator at 37°C with 5% carbon dioxide for 2 h. The excess extract in the wells was discarded. 2 mL of 1% BSA-PBS solution was added, and incubation was performed in an incubator at 37°C with 5% carbon dioxide for 1 h. The liquid in the wells was discarded, and the wells were washed three times with PBS. The liquid in the wells was discarded, and the plate was sealed with a sealing film and stored at 4°C for later use.

Seeding: Cells were seeded into 6-well plates at a seeding density of 1.4E5 cells/well and incubated in an incubator (37°C, 5% $CO_2$) overnight (24 h).

Scratching: Grouping was performed according to the experimental design, with three replicates per group. Culture was continued for 24 h in an incubator (37°C, 5% $CO_2$). When the cell confluence in the 6-well plates reached 90% or more, scratching was performed. Two vertical scratches (baselines) were created longitudinally in each well of the 6-well plate using a 10 μL pipette tip (tip perpendicular to ruler edge), maintaining 2 cm spacing between scratches; horizontal scratches were then made perpendicular to the baselines near the central axis of each well of the 6-well plate; uniform pressure was applied during scratching with the tip to ensure consistent scratch width.

Two vertical scratches (baselines) were created longitudinally in each well of the 6-well plate using a 10 μL pipette tip (tip perpendicular to ruler edge) maintaining 2 cm spacing between scratches;
horizontal scratches were then made perpendicular to the baselines near the central axis of each well of the 6-well plate; uniform pressure was applied during scratching with the tip to ensure consistent scratch width.

Washing: After scratching, the cells were gently washed 3 times by adding 1 mL of PBS solution to each well to remove detached cells caused by scratching. After washing, 2 mL of serum-free culture medium was added to each well, and culture was continued in an incubator at 37°C with 5% $CO_2$.

Image acquisition: At 0 h post-scratching, image acquisition was performed at 4× magnification. At 24 h post-scratching, washing was performed with PBS once, followed by image acquisition at 4× magnification. If no obvious migration was observed, at 48 h post-scratching, washing was performed with PBS once, followed by image acquisition at 4× magnification. The observation area was defined as the scratch region between two intersection points of the vertical baselines and horizontal scratch, and observation was performed from left to right. At 0 h, 9 images of the typical areas were taken; at 24 h and 48 h, 9 consecutive images were taken (the detached cells were removed by PBS washing before image acquisition at 24 h and 48 h). The results were as shown in FIG. 13, indicating that the composition significantly promoted osteoblast migration.

**Cell proliferation:** Cell culture: Cells were digested and collected, and then counted using a cell counting plate. The seeding density of the cells was adjusted to 10000 cells/mL.

Addition of cell suspension: The composition was soaked in normal culture medium for 2 h, and then the culture medium was discarded. 200 μL of the cell suspension was gently added from the top of the sterile sample (two parallel

experiments were performed, with sample 1 (2#-1) and sample 2 (2#-2) both being products obtained in Example 1) until the cell suspension was completely absorbed into the sample. The culture dishes containing the samples were placed in a cell incubator.

Culture: After 6 hours, a small amount of culture medium was gently added around the sample until the sample was submerged. After 16 hours, 1-2 ml of culture medium was slowly added into the culture dish. Culture was performed for 1 day, 3 days, 5 days, 7 days, and 9 days, respectively (with culture medium replacement every other day).

Detection: After the culture was completed, the culture medium was removed, washing was performed 2-3 times with PBS, the cells were digested using 0.25% trypsin for cell counting, and the results were averaged. The results were as shown in FIG. 14, indicating that the composition significantly promoted osteoblast proliferation.

**Cell adhesion:** Cell culture: MC3T3-E1 cells at passage 2 to 3 were used. When the cell confluence reached 80%, the cells were digested and collected, and counted using a cell counting plate. The seeding density of the cells was adjusted to $2 \times 10^7$ cells/mL.

Addition of cell suspension: The composition was soaked in normal culture medium for 2 h, and then the medium was discarded. 500 $\mu$L of the cell suspension ($4 \times 10^6$ cells) was gently added in three aliquots from the center of the sterile sample until the cell suspension was completely absorbed into the sample. During the operation, care was taken to prevent the cell suspension from falling into the well plate.

Incubation and culture: After 6 hours, a small amount of culture medium was gently added around the sample until the sample was submerged. After 16 hours, 1-2 ml of culture medium was slowly added into the culture dish, and culture was performed for 2 h and 4 h, respectively.

Detection: After the culture was completed, 2 osteoblast/composition co-culture samples were randomly selected, along with 2 blank composition samples (without seeded cells). The culture medium was removed, and the samples were gently washed 2 to 3 times with PBS under gentle shaking, fixed with 3% glutaraldehyde for 30 minutes, then washed 2 to 3 times with PBS, gradually dehydrated with an ethanol concentration gradient series (30%, 50%, 70%, 90%, and 100%) with 2-minute soaking and washing at each concentration. After vacuum drying and sputter-coating with gold, the samples were observed under a scanning electron microscope to examine the cell adhesion morphology on the material surface at different time points. The results were as shown in FIG. 15, demonstrating that osteoblasts could adhere to the composition and form a lamellar structure, which facilitated the bone repair process.

***In vitro* degradation assay:** The sample was cut into four uniform pieces. Then, these four pieces and filter-sterilized 0.01 mol PBS buffer (pH = 7.4) were added into sterile centrifuge tubes at a ratio of $m_{product}$: $V_{PBS}$ = 1 g/200 ml. After gentle shaking to ensure full contact between the material and PBS solution, degradation was simulated in a constant-temperature incubator or water bath at 37°C. Residual samples were collected at 1 d, 2 d, 4 d, 9 d, 14 d, 17 d, 20 d, and 30 d, and the degradation rate was calculated using the constant-weight method. The results were as shown in FIG. 16, indicating that the cancellous bone material exhibited good degradability.

In summary, the above description is merely preferred examples of the present disclosure, and is not intended to limit the present disclosure in any other form. Those skilled in the art may use the disclosed technical content to make changes or modifications, which are equivalent embodiments. However, any simple amendments, equivalent variations and modifications made to the above examples on the basis of the technical substance of the present disclosure, without departing from the content of the technical solutions of the present disclosure, all still fall within the scope of protection of the present disclosure.

## Claims

**1.** A composition for use in the preparation of a bone repair material, wherein, based on 100% by mass of the total dry matter of the composition, the composition comprises:

70%-90% of coralline hydroxyapatite particles,
5%-30% of a recombinant human collagen, and
0%-7% of an excipient;
wherein the bone repair material is a cancellous bone-mimicking material and has a porosity of 85%-99%.

**2.** The composition for use according to claim 1, wherein the bone repair material includes a bone repair material used for filling and/or repair of bone defects.

**3.** The composition for use according to claim 1, wherein the bone repair material includes a bone repair material used in dentistry, orthopedics, neurosurgery, or plastic surgery;

preferably, the bone repair material used in dentistry includes a bone repair material for extraction socket filling

after tooth extraction or residual root extraction, alveolar ridge restoration, repair of alveolar bone defects caused by periodontal diseases, filling and restoration of dentoalveolar bone defects or bone volume deficiency, or filling of bone defects in maxillofacial non-load-bearing areas;
preferably, the bone repair material used in orthopedics includes a bone repair material used for repair of bone defects in non-load-bearing areas, and more preferably includes a bone repair material used in fractures accompanied by bone defects, bone nonunion or malunion and orthopedic correction, benign cystic bone lesions, spinal fusion caused by lumbar instability or lumbar canal stenosis, or bone grafting in joint arthrodesis;
preferably, the bone repair material used in neurosurgery includes a bone repair material used for filling of cranial defects caused by craniotomy drilling, filling of cranial defects caused by decompressive craniectomy, filling of bone gaps caused by craniectomy milling cutter, or repair of cranial defects caused by intraoperative cranial bone removal;
preferably, the bone repair material used in plastic surgery includes a bone repair material used for filling and/or repair of bone defects.

**4.** The composition for use according to claim 1, wherein the composition is a solid porous material formed by coralline hydroxyapatite particles bonded to each other through a recombinant human collagen.

**5.** The composition for use according to claim 1, wherein the coralline hydroxyapatite particles have a particle size of 0.1 mm-2 mm, a pore size of 50 μm-800 μm, and a porosity of 50%-90%.

**6.** The composition for use according to claim 1, wherein the conversion rate of the coralline hydroxyapatite particles is 5%-80%, more preferably 5%-30%.

**7.** The composition for use according to claim 1, wherein the excipient comprises one of or a combination of two or more of cross-linked porous starch, sodium carboxymethyl cellulose, chitosan, carboxymethyl chitosan, and hydroxypropyl methyl cellulose.

**8.** The composition for use according to claim 1, wherein the coralline hydroxyapatite particles are prepared by soaking coral stone in a cutting protective agent, performing crushing and granulating, and performing hydrothermal exchange;

wherein the cutting protective agent is a solution containing a polyol;
preferably, the polyol is selected from glycerol, ethylene glycol, sorbitol, and butanediol;
preferably, based on the total mass of the cutting protective agent, the mass fraction of the polyol is ≥ 20%;
preferably, the duration of the soaking is ≥ 3 h.

**9.** The composition for use according to claim 8, wherein the raw material of the coral stone comprises natural coral and/or artificially cultivated coral;

preferably, the natural coral includes *Porites* and/or *Goniopora,* more preferably *Porites;*
preferably, the hydrothermal exchange comprises: immersion in a saturated diammonium hydrogen phosphate solution, and a reaction at 0.1-3 MPa and 150°C-220°C for 6-19 h.

**10.** The composition for use according to claim 1, wherein the composition is in the form of granules, blocks, flakes, or powder.

FIG. 1

200 μm
EHT = 10.00 kV
WD = 9.8 mm
Signal A = SE2
Mag = 50 X
Date: 28 Sep 2024

FIG. 2

FIG. 3

a
b
c
a
b
c

FIG. 4

FIG. 5

a. Plug-shaped sample of Example 1
b. Cross-section of plug-shaped sample of Example 1
c. Cancellous bone-mimicking material of Example 3
d. Cancellous bone-mimicking material of Example 4
e. Cubic sample of Example 5
f. Disc-shaped sample of Example 6
g. Cancellous bone-mimicking material of Example 8
h. Cancellous bone-mimicking material of Example 9
i. Cancellous bone-mimicking material of Comparative example 1
j. Cancellous bone-mimicking material of Comparative example 2
k. Cancellous bone-mimicking material of Comparative example 3
l. Cancellous bone-mimicking material of Comparative example 4
m. Cancellous bone-mimicking material of Comparative example 5

FIG. 6

a
b
c
d
e

FIG. 7

a
b
c
d

FIG. 8

a

b

FIG. 9

FIG. 10

a
b
c
d
e
f

FIG. 11

Relative viability (%)
150
100
50
0
**
*
*
**
SC
3.13%
6.25%
12.50%
25.00%
50%
100%
PC
Experimental groups (stock solution dilution)

FIG. 12

0h
24h
48h
SC
2#

FIG. 13

OD450
0.800
0.700
0.600
0.500
0.400
0.300
0.200
0.100
0.000
1d
3d
5d
7d
9d
Time d
2#-2
2#-1
2#-1
2#-2

FIG. 14

10 μm
EHT = 5.00 kV
WD = 8.4 mm
Signal A = SE2
Mag = 1.00 K X
Width = 114.3 μm
Date: 29 Oct 2024
ZEISS

FIG. 15

30.000
20.000
10.000
0.000
1d
2d
4d
9d
14d
17d
20d
30d
Degradation rate%

FIG. 16

Autologous control
Experimental group 1
30d
Experimental group 1
60d

FIG. 17

Experimental group 1
Control group (similar product)
Experimental group 2
Blank control group
0d
30d
60d
90d

FIG. 18

A. Material implantation 0W B. Material implantation 12W C. Material implantation 20W

FIG. 19

A. Residual root extraction, material implantation 0W

B. Material implantation 14W, osteogenic imaging

C. Material implantation 16W, implant imaging

FIG. 20

Europäisches
Patentamt
European
Patent Office
Office européen
des brevets

# EUROPEAN SEARCH REPORT

Application Number
EP 25 20 5883

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2017/304502 A1 (VICKERS SCOTT M [US] ET AL) 26 October 2017 (2017-10-26)<br>* paragraphs [0053], [0070]; claims 4,11,13 * | 1-10 | INV.<br>A61L27/46<br>A61L27/56 |
| A | CN 116 510 077 A (JIANGSU JINGSEN BIOLOGICAL MEDICINE NEW MATERIAL TECH CO LTD) 1 August 2023 (2023-08-01)<br>* claims 1,2,5,8 * | 1-10 | |
| A | US 2017/119933 A1 (MCKAY WILLIAM F [US]) 4 May 2017 (2017-05-04)<br>* paragraphs [0059], [0062], [0067]; claim 8 * | 1-10 | |
| X | CN 107 952 115 A (BEIJING PASHION BIOTECH CO LTD) 24 April 2018 (2018-04-24)<br>* claims 1,2,5; example 2 * | 1-10 | |

TECHNICAL FIELDS SEARCHED (IPC)

A61L

The present search report has been drawn up for all claims

| Place of search | Date of completion of the search | Examiner |
|---|---|---|
| The Hague | 27 February 2026 | Siebum, Bastiaan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2
EPO FORM 1503 03.82 (P04C01)

## ANNEX TO THE EUROPEAN SEARCH REPORT ON EUROPEAN PATENT APPLICATION NO.

EP 25 20 5883

This annex lists the patent family members relating to the patent documents cited in the above-mentioned European search report. The members are as contained in the European Patent Office EDP file on The European Patent Office is in no way liable for these particulars which are merely given for the purpose of information.

27-02-2026

| Patent document cited in search report | Publication date | Patent family member(s) | Publication date |
|---|---|---|---|
| US 2017304502 A1 | 26-10-2017 | AU 2017252435 A1 | 04-10-2018 |
| | | EP 3445413 A1 | 27-02-2019 |
| | | KR 20180128482 A | 03-12-2018 |
| | | US 2017304502 A1 | 26-10-2017 |
| | | US 2020289711 A1 | 17-09-2020 |
| | | WO 2017184959 A1 | 26-10-2017 |
| CN 116510077 A | 01-08-2023 | NONE | |
| US 2017119933 A1 | 04-05-2017 | EP 3368095 A1 | 05-09-2018 |
| | | US 2017119933 A1 | 04-05-2017 |
| | | WO 2017074894 A1 | 04-05-2017 |
| CN 107952115 A | 24-04-2018 | CN 107952115 A | 24-04-2018 |
| | | WO 2018072679 A1 | 26-04-2018 |
| | | ZA 201903065 B | 30-09-2020 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 108070032 B